# EUROPEAN PATENT SPECIFICATION

(11) **EP 4 489 973 B1**
(45) Date of publication and mention of the grant of the patent: **26.08.2026**
(21) Application number: 23708451.2
(22) Date of filing: 27.02.2023
(51) Int. Cl.: B41J 29/02, A61B 5/338, A61B 5/344

(54) **PRINTING MECHANISM FOR MEDICAL MONITORING DEVICE AND MEDICAL MONITORING DEVICE**
DRUCKMECHANISMUS FÜR MEDIZINISCHE ÜBERWACHUNGSVORRICHTUNG UND MEDIZINISCHE ÜBERWACHUNGSVORRICHTUNG
MÉCANISME D'IMPRESSION POUR DISPOSITIF DE SURVEILLANCE MÉDICALE ET DISPOSITIF DE SURVEILLANCE MÉDICALE

(30) Priority: 07.03.2022 CN 202210216454; 07.03.2022 CN 202220478656 U
(43) Date of publication of application: 15.01.2025
(73) Proprietor: Koninklijke Philips N.V., 5656 AE Eindhoven (NL)
(72) Inventor: YANG, Lei, 5656 AG Eindhoven (NL); TANG, Liang Yong, 5656 AG Eindhoven (NL)
(74) Representative: Philips Intellectual Property & Standards
(86) International application number: PCT/EP2023/054765
(87) International publication number: WO 2023/169846

(56) References cited:
- EP-A1- 2 853 405
- US-A- 6 106 178
- US-A1- 2001 017 644
- US-A1- 2003 081 083
- US-A1- 2009 073 213
- US-A1- 2012 193 864
- US-A1- 2020 101 273

## Description

### FIELD OF THE INVENTION

The present invention relates to a printing mechanism for a medical monitoring device and a medical monitoring device comprising such a printing mechanism.

### BACKGROUND OF THE INVENTION

A medical monitoring device such as a fetal monitor, an electrocardiograph, a defibrillator, or an ultrasound diagnostic device generally comprises a main unit and a printing mechanism disposed in the main unit. In the case of the printing mechanism for a fetal monitor, for example, the laminated thermal print papers are usually housed in a print paper drawer. When the printing mechanism operates, a rubber platen driven by a stepper motor rotates at a preset speed to drag a print paper past a thermal printing head to record a curve characterizing fetal vital characteristics on thermal print paper. The medical monitoring device may operate in a horizontally arranged state but in some cases the medical monitoring device needs to operate in a vertically hung state. If no action is taken when the medical monitoring device operates in the vertically hung state, the laminated thermal print papers that meet a standard for the medical monitoring devices such as a fetal monitor or the like may tilt as a whole, which would affect the smoothness in feeding the print paper by thermal printing head and lead to paper jams in more severe cases.

Therefore, there is a need to improve the existing printing mechanism for the medical monitoring device. Document US 6106178 A discloses a printer and a paper tray for a printer and represents relevant prior art for the present invention.

### SUMMARY OF THE INVENTION

It is an object of the present invention to provide a printing mechanism for a medical monitoring device which can overcome at least one of the above-mentioned disadvantages in the prior art.

According to the present invention, there is provided a printing mechanism for a medical monitoring device comprising:
a movable frame assembly comprising a frame; and
a print paper drawer for holding the print papers, the print paper drawer being mounted on the frame to move along with the movable frame assembly;
wherein a front end wall of the print paper drawer is formed as a curved wall comprising a first portion extending perpendicularly from a front end of a bottom plate of the print paper drawer and a second portion extending from the first portion and bending towards a rear end of the print paper drawer with respect to the first portion, and the second portion forms an anti-tilting surface for preventing the print papers from tilting.

Preferably, the second portion may be bent at an angle of 20° to 25° with respect to the first portion.

Preferably, the front end wall may comprise front end wall portions that extend from a first sidewall and a second sidewall of the print paper drawer respectively so that a space is formed between two front end wall portions.

Preferably, the print paper drawer may further comprise a loading ramp extending outwardly from a top edge of the first portion, and the loading ramp is formed as an arcuate surface that gradually lowers toward the bottom plate of the print paper drawer.

Preferably, the printing mechanism may further comprise a paper lifting mechanism comprising a shaft rotatably provided at a front end of the print paper drawer, a lifting bar provided on the shaft to rotate together with the shaft, and an operating handle attached to the shaft to drive the shaft to rotate, and the lifting bar is accommodated in an opening in the bottom plate or can rotate along with the shaft to pass through the opening in the bottom plate.

Preferably, the paper lifting mechanism may further comprise a reset spring which is attached to the operating handle to cause the operating handle to turn back to an initial position.

Preferably, a silicone sleeve may be provided at the end of the lifting bar.

Preferably, the printing mechanism may further comprise:
a support plate;
a support arm disposed on the support plate, the support arm provided with a printing head thereon, the movable frame assembly further comprising a rubber platen mounted at a front end of the frame adjacent to the outside of the medical monitoring device to be in contact with the printing head under pressure; and
a driving mechanism for driving the rubber platen to rotate;
wherein the movable frame assembly is movably disposed on the support plate and between the support plate and the support arm.

Preferably, the printing mechanism may further comprise a locking mechanism comprising a pair of resilient clamping claws disposed opposite to each other at a rear end of the frame and a locking post disposed on the support plate correspondingly to the resilient clamping claws, the resilient clamping claws clamp together, the resilient clamping claws open under an external pushing force to receive the locking post and clamp the locking post tightly, and the resilient clamping claws are disengaged from the locking post under an external pulling force.

Preferably, the driving mechanism may comprise a first pulley fixedly mounted at one end of the rubber platen, an electric motor mounted on the frame, a second pulley mounted on a rotating shaft of the electric motor, and a timing belt for connecting the first pulley and the second pulley so as to drive the rubber platen to rotate when the electric motor operates, and the electric motor is disposed at a rear end of the frame adjacent to the inside of the medical monitoring device.

Preferably, the support arm is L-shaped, the support arm is rotatably mounted on the support plate at one end and provided with a printing head mounting portion at the other end, and the printing head is mounted on the printing head mounting portion.

Preferably, the frame may comprise a first mounting plate and a second mounting plate disposed opposite to each other, and a first connecting plate and a second connecting plate connecting fixedly the first mounting plate and the second mounting plate together, a first guide slot extending along a longitudinal direction is formed in the first mounting plate and a second guide slot extending along the longitudinal direction is formed in the second mounting plate, and two ends of the printing head mounting portion are movably supported in the first guide slot and the second guide slot.

Preferably, the medical monitoring device may be a fetal monitor.

According to another aspect, there is provided a medical monitoring device, wherein the medical monitoring device comprises a printing mechanism for a medical monitoring device as above stated.

In the printing mechanism for the medical monitoring device according to the present invention an anti-tilting surface for preventing the print papers from tilting is disposed at the front end of the print paper drawer, even if the medical monitoring device operates in a vertically hung state, the end of the print papers contacting the anti-tilting surface does not slip and tilt during printing while the smoothness in feeding the print paper to the printing head is not affected, thereby ensuring normal operation of the printing mechanism.

### BRIEF DESCRIPTION OF THE DRAWINGS

FIG. 1 schematically shows in a perspective view a printing mechanism for a medical monitoring device according to the present invention;
FIG. 2 shows schematically in a perspective view a movable frame assembly of a printing mechanism for a medical monitoring device according to the present invention, wherein an electric motor and a rubber platen are mounted on the frame;
FIG. 3 shows schematically in a perspective view the printing mechanism for the medical monitoring device according to the present invention, wherein a support arm, a printing head mounting portion and a rubber platen have been removed to clearly show a print paper drawer;
FIG. 4 is a top view of the structure shown in FIG. 3;
FIG. 5 is a partially enlarged view of FIG. 4 showing a locking post to be clamped by resilient clamping claws;
FIG. 6 is a partially enlarged view of FIG. 4 showing a locking post clamped by resilient clamping claws in a locked state;
FIG. 7 is a cross-sectional view showing the structure of the printing mechanism;
FIG. 8 is a perspective view showing a print paper drawer, wherein a paper lifting mechanism is mounted on the print paper drawer;
FIG. 9 is another perspective view showing a print paper drawer, wherein the paper lifting mechanism is removed from the print paper drawer;
FIG. 10 is a cross-sectional view taken along a line 10-10 of FIG. 9; and
FIG. 11 is another perspective view showing a print paper drawer as viewed from the bottom, wherein a paper lifting mechanism is mounted on the print paper drawer.

### DETAILED DESCRIPTION OF THE EXEMPLARY EMBODIMENTS

Preferred embodiments of the present invention are described in detail hereinafter in connection with the drawings. It should be understood by those skilled in the art that the drawings are intended to illustrate the invention only and are not meant to form any limit to the present invention.

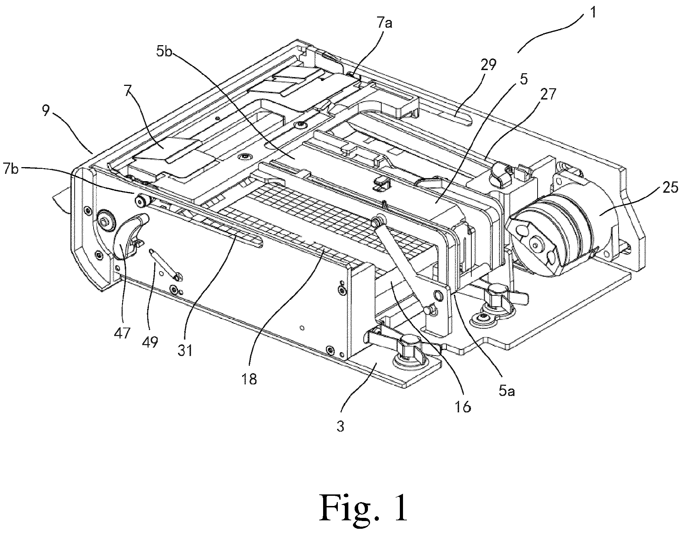

FIG. 1 schematically shows in a perspective view a printing mechanism for a medical monitoring device according to the present invention. As shown in FIG. 1, a printing mechanism 1, for a medical monitoring device according to the present invention, generally comprises a support plate (or a base plate) 3, which is typically mounted on a housing of the medical monitoring device (not shown) such as a fetal monitor. The printing mechanism 1, for the medical monitoring device according to the present invention, further comprises a support arm 5. In a preferred embodiment, the support arm 5 is substantially L-shaped, one end portion 5a of the support arm 5 is rotatably mounted on the support plate 3, and the other end 5b of the support arm 5 is provided with a printing head mounting portion 7 on which a printing head for example a thermal printing head is mounted. In Fig 1, the printing head is invisible because it is mounted on the underside of the printing head mounting portion 7. The printing head is electrically communicated with a main unit of the medical monitoring device by wire or wirelessly, and thus receives signals from the main unit to perform the printing operations. The printing mechanism 1, for the medical monitoring device according to the present invention, also comprises a movable frame assembly 9, which is movably disposed on the support plate 3 and in a space between the support plate 3 and the support arm 5.

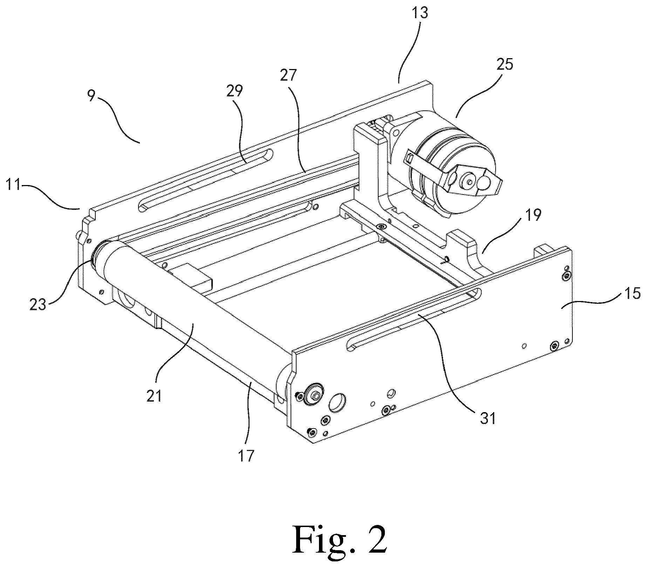

FIG. 2 shows schematically in a perspective view a movable frame assembly of a printing mechanism for a medical monitoring device according to the present invention, wherein an electric motor and a rubber platen are mounted on the frame. As shown in FIG. 2, the movable frame assembly 9 comprises a frame 11. The frame 11 comprises a first mounting plate 13 and a second mounting plate 15 disposed opposite to each other, and a first connecting plate 17 and a second connecting plate 19 that fixedly connect the first mounting plate 13 and the second mounting plate 15 together. In the preferred embodiment, the first mounting plate 13, the second mounting plate 15, the first connecting plate 17, and the second connecting plate 19 are metal members such as aluminum or other lightweight metal or alloy members, which are formed separately and then connected together by fasteners such as screws so that the frame 11 is formed as a highly rigid metal frame. However, it should be understood that the first mounting plate 13, the second mounting plate 15, the first connecting plate 17, and the second connecting plate 19 can also be cast in one piece from metal. Of course, the first mounting plate 13, the second mounting plate 15, the first connecting plate 17, and the second connecting plate 19 can also be molded in one piece from plastic. The movable frame assembly 9 also comprises a print paper drawer 16 mounted between the first mounting plate 13 and the second mounting plate 15 to hold the print papers 18. In FIG. 2, the print paper drawer 16 mounted on the frame 11 is not shown.

The movable frame assembly 9 also comprises a rubber platen 21 whose ends are rotatably supported on the first mounting plate 13 and the second mounting plate 15. The rubber platen 21 is rotatably supported at the front end of the first mounting plate 13 and the second mounting plate 15 adjacent to the outside of the medical monitoring device by bearings, and a first pulley 23 is fixedly mounted at one end of the rubber platen 21. The movable frame assembly 9 also comprises an electric motor 25, which is spaced apart from the rubber platen 21 along a longitudinal direction of the first mounting plate 13 and mounted on the first mounting plate 13, and a second pulley (invisible in the drawings) is mounted on a rotating shaft of the electric motor 25. A timing belt 27 connects the first pulley 23 and the second pulley so that the electric motor drives the rubber platen 21 to rotate by means of the second pulley, the timing belt 27, and the first pulley 23 when the electric motor operates. Thus, according to the present invention, the rubber platen 21, the electric motor 25, and the timing belt 27 connecting the rubber platen 21, and the electric motor 25 are all mounted on the frame 11. In this way, even though the movable frame assembly 9 is pulled out of the housing of the medical monitoring device to pick up and add the print paper and then is inserted into the housing of the medical monitoring device 13, the rubber platen 21, the electric motor 25, and the timing belt 27 move together with the frame but their relative positions remain unchanged, thus ensuring that they are positioned accurately, which in turn allows the printing mechanism to operate smoothly without generating noise or generating significantly reduced noise.

Preferably, the electric motor 25 is disposed at a rear end of the first mounting plate 13 adjacent to the inside of the medical monitoring device, which allows the movable frame assembly 9 to be formed with a substantially uniform thickness and thus have a relatively flat and regular shape. As a result, the printing mechanism may have a relatively compact and regular layout. More preferably, the first pulley 23, the second pulley, and the electric motor 25 are all provided adjacent to the first mounting plate 13, thus making the overall structure more compact and occupying as little space as possible. The first mounting plate 13 and the second mounting plate 15 are sized such that the rubber platen 21, the first pulley on the rubber platen 21, the timing belt 27, the electric motor 25, and the second pulley on the electric motor 25 are all located inside of at least one of the first mounting plate 13 and the second mounting plate 15, ensuring that no moving component protrudes out of the frame 11 or is exposed to the outside of the frame 11. Although the first mounting plate 13 and the second mounting plate 15 may have the same length, in the preferred embodiment the length of the second mounting plate 15 may be shorter than that of the first mounting plate 13 because the second mounting plate 15 does not have to support the electric motor 25, which reduces the amount of material used and lowers the weight of the frame 11. Although in the above preferred embodiment the driving mechanism for driving the rubber platen to rotate comprises the electric motor, the first pulley, the second pulley, and the timing belt, it should be understood that the driving mechanism for driving the rubber platen to rotate may be of other construction, including for example an electric motor mounted on the frame, which drives the rubber platen also mounted on the frame by gear engagement.

A first guide slot 29 and a second guide slot 31 extending along the longitudinal direction are formed in the first mounting plate 13 and the second mounting plate 15 respectively. Two ends 7a and 7b of the printing head mounting portion 7 are movably supported in the first guide slot 29 and the second guide slot 31 respectively.

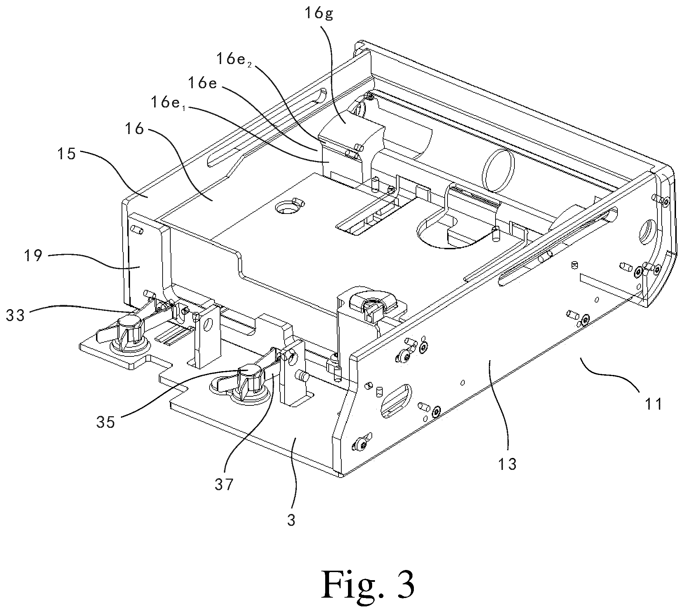

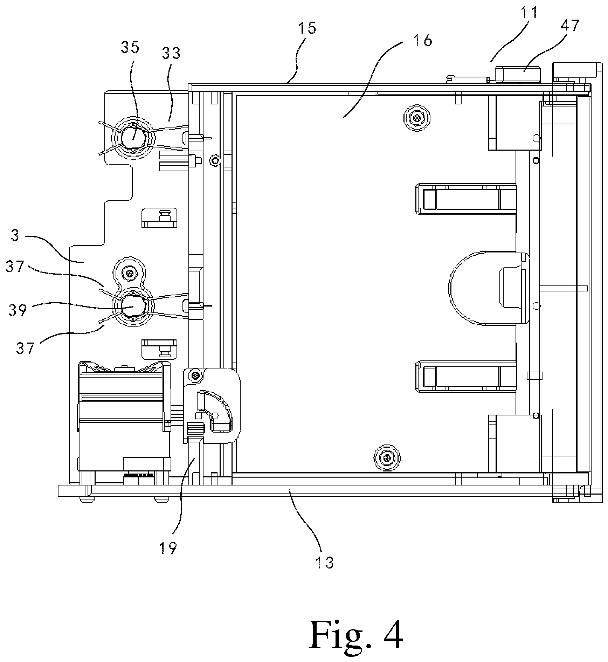

FIG. 3 shows schematically in a perspective view the printing mechanism for the medical monitoring device according to the present invention, wherein a support arm, a printing head mounting portion and a rubber platen have been removed to clearly show a print paper drawer, FIG. 4 is a top view of the structure shown in FIG. 3. A print paper drawer 16 for holding the print papers 18 is clearly shown in FIG. 3. The print paper drawer 16 may be molded from plastic, and the print paper drawer 16 is fixedly supported on the frame 11, for example, by snap, screw, or other fastening means and may move along with the frame 11 or the movable frame assembly 9.

The printing mechanism 1 for the medical monitoring device according to the present invention further comprises a locking mechanism comprising a first locking member 33 provided on the outside of the second connecting plate 19 of the frame 11 (i.e., at a rear side of the frame 11 facing the inside of the housing of the medical monitoring device), and a second locking member 35 provided on the support plate 3. When the frame 11 provided with the print paper drawer 16 is pushed into the housing of the medical monitoring device, the first locking member 33 and the second locking member 35 can be engaged together under external pushing force, thereby locking the movable frame assembly 9 and the print paper drawer 16 in place in the housing of the medical monitoring device. When the frame 11 provided with the print paper drawer 16 is pulled outwardly from the housing of the medical monitoring device, the first locking member 33 and the second locking member 35 may be disengaged from each other under external pulling force, thereby pulling the movable frame assembly 9 and the print paper drawer 16 outwardly from the housing of the medical monitoring device.

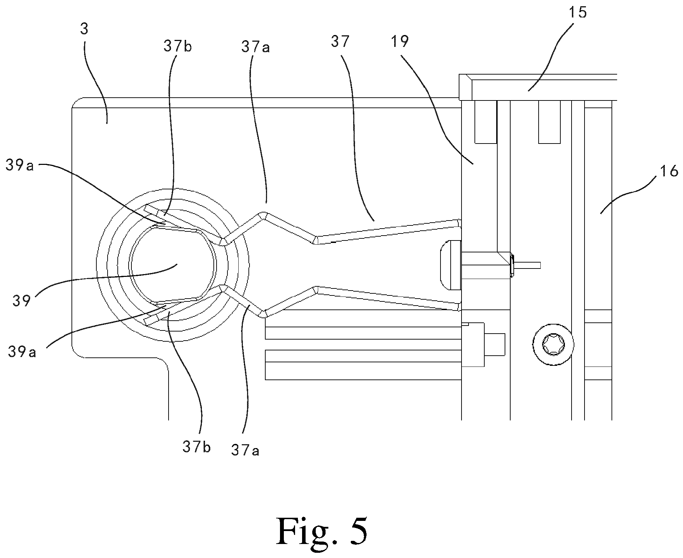

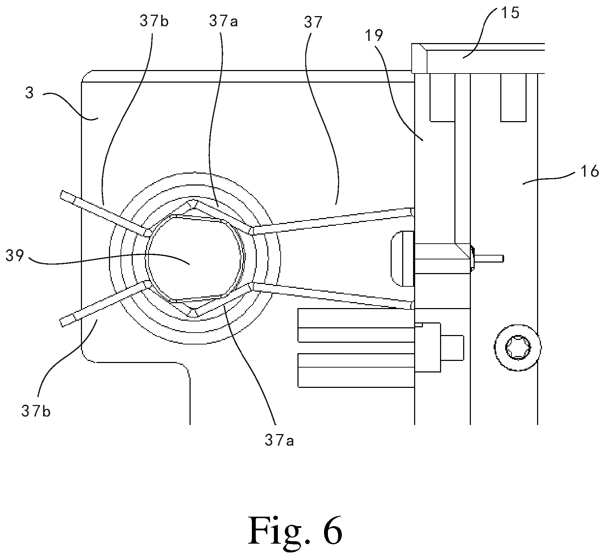

In the preferred embodiment as shown in FIGS. 3 and 4, the first locking member 33 comprises a pair of resilient clamping claws 37 disposed opposite to each other, and the resilient clamping claws 37 clamp together. The second locking member 35 comprises a locking post 39 disposed on the support plate 3 correspondingly to the resilient clamping claws 37. When the frame 11 provided with the print paper drawer 16 is pushed into the housing of the medical monitoring device, the resilient clamping claws 37 open under an external pushing force to receive the locking post 39 and subsequently clamp the locking post 39 tightly, thereby locking the print paper drawer 16 in place in the housing of the medical monitoring device, as shown in FIGS. 5 and 6. When the frame 11 provided with the print paper drawer 16 is pulled outwardly from the housing of the medical monitoring device, the resilient clamping claws 37 are disengaged from the locking post 39 under an external pulling force, thereby pulling the print paper drawer 16 outwardly from the housing of the medical monitoring device.

In order to reliably clamp the locking post 39 by the pair of resilient clamping claws 37, each of the resilient clamping claws 37 is formed with a wrapping portion 37a which is bent outwardly and subsequently bent inwardly. Two oppositely arranged wrapping portions 37a preferably form a "()" or "< >" shapes to hold the locking post 39. Further, each of the resilient clamping claws 37 may further comprises a guiding portion 37b that is bent outwardly again from the wrapping portion 37a. Two oppositely arranged guiding portions 37b are preferably in a trumpet shape to guide the locking post 39 into a space between the two wrapping portions 37a. Although the locking post 39 may be in a cylindrical shape, two sides of the locking post 39 are preferably beveled and two planes 39a formed by beveled sides converge toward the print paper drawer 16, which further helps to guide the locking post 39 into engagement with the pair of resilient clamping claws 37.

According to the locking mechanism of the preferred embodiment of the present invention, when the print paper drawer 16 is about to be fully closed, the inner walls of the wrapping portions 37a of the resilient clamping claws 37 exert a pushing force on the locking post 39, which helps to push the print paper drawer 16 into the housing. After the print paper drawer 16 is fully closed, the resilient clamping claws 37 reset and generate a "click" sound to provide feedback to the user that the print paper drawer 16 is fully closed.

In the preferred embodiment shown in FIGS. 3 and 4, there are two pairs of resilient clamping claws 37 and two corresponding locking posts 39. It is understood that it is possible to have only one or more pairs of resilient clamping claws 37 and corresponding locking post 39. It is also feasible that the pairs of resilient clamping claws are disposed on the support plate 3 while the locking posts 39 are disposed on the frame 11.

In a normal state, the movable frame assembly 9 is inserted into the space between the support plate 3 and the support arm 5 in the housing of the medical monitoring device such that the printing head on the printing head mounting portion 7 is in contact with the rubber platen 21 under pressure. The print paper 16 may pass between the rubber platen 21 and the printing head. When the printing mechanism operates, the electric motor 25 drives the rubber platen 21 to rotate by means of the second pulley, the electric motor 25, and the first pulley 23, thereby dragging thermal print paper past the printing head to record a curve characterizing fetal vital characteristics on the print paper.

When the movable frame assembly 9 is pulled outwardly to pick up and add the print papers, the resilient clamping claws 37 disengage from the locking posts 39 under external pulling force, thereby moving the print paper drawer to the outside of the housing of the medical monitoring device to pick up and add the print paper. After picking up and adding the print paper, the resilient clamping claws 37 open under external pushing force to receive the locking post 39 and subsequently clamp the locking post 39 tightly, thereby locking the print paper drawer 16 in place in the housing of the medical monitoring device.

The printing mechanism according to the present invention has fewer parts and is simpler in design and assembly. And, in use, the user may perform the opening and closing operation of the print paper drawer with only one hand without any additional pressing operation or great effort. Further, the print paper drawer may be pushed or pulled steadily without any wobbling and achieve a stable and reliable locking.

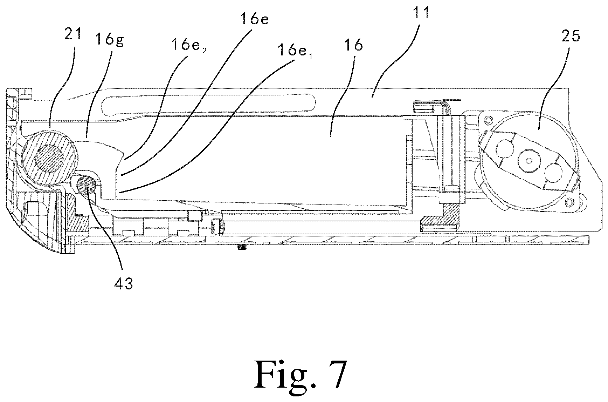

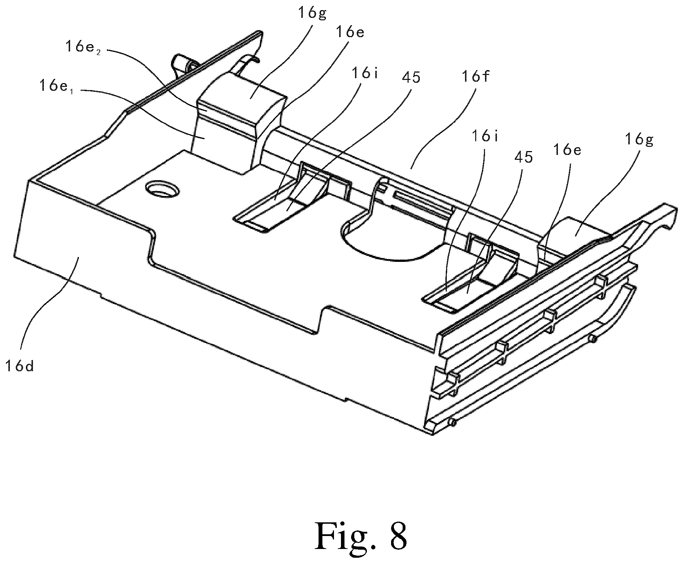

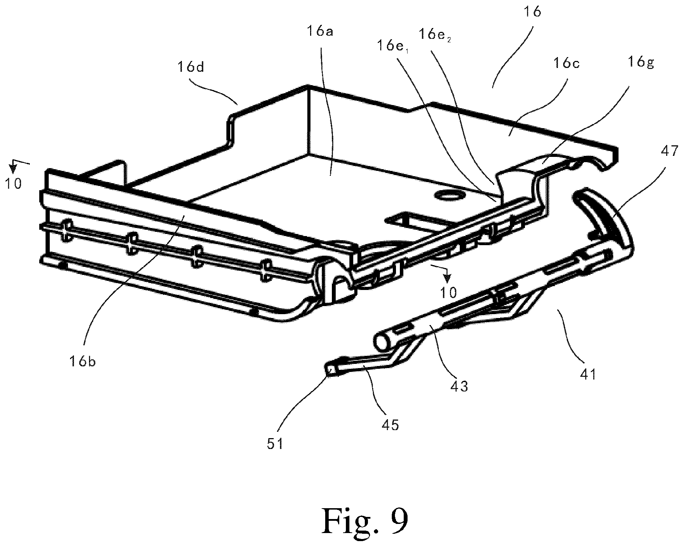

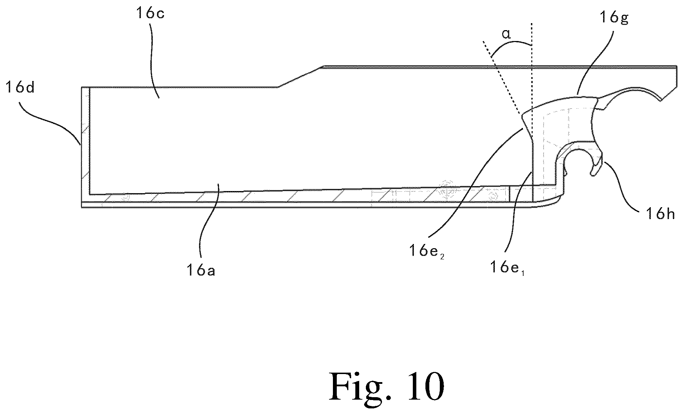

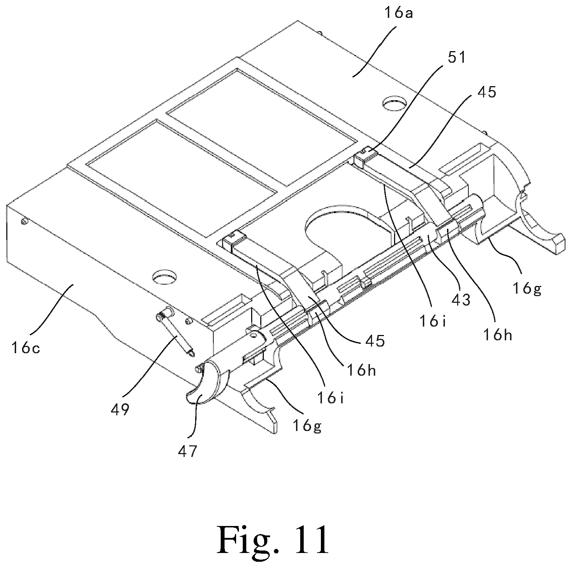

FIG. 7 is a cross-sectional view showing the structure of the printing mechanism; FIG. 8 is a perspective view showing a print paper drawer, wherein a paper lifting mechanism is mounted on the print paper drawer; FIG. 9 is another perspective view showing a print paper drawer, wherein the paper lifting mechanism is removed from the print paper drawer; FIG. 10 is a cross-sectional view taken along a line 10-10 of FIG. 9; and FIG. 11 is another perspective view showing a print paper drawer as viewed from the bottom, wherein a paper lifting mechanism is mounted on the print paper drawer. The print paper drawer 16 may be molded from plastic. As shown in FIGS. 3, 4, and 7 and as described above, the print paper drawer 16 is fixedly supported on the frame 11, for example, by snaps, screws, or other fastening means, and may move along with the frame 11 or the movable frame assembly 9.

As shown in FIGS. 8-11, the print paper drawer 16 is substantially in a drawer shape and comprises a bottom plate 16a, a first side wall 16b, and a second side wall 16c extending perpendicularly from two sides of the bottom plate 16a respectively, a rear end wall 16d extending perpendicularly from the rear end of the bottom plate 16a, and a front end wall 16e formed at the front end of the bottom plate 16a. The rear end of the bottom plate 16a refers to an end of the bottom plate 16a facing the inside of the housing of the medical monitoring device. The rear end of the bottom plate 16a refers to an end of the bottom plate 16a facing the outside of the housing of the medical monitoring device, i.e., the end adjacent to the rubber platen 21. According to the present invention, the front end wall 16e of the print paper drawer 16 is not a straight wall but a curved wall. The curved wall comprises a first portion 16e₁ extending perpendicularly from the front end of the bottom plate 16a and a second portion 16e₂ extending from the first portion 16e₁ and bending towards the rear end with respect to the first portion 16e₁. The second portion 16e₂ forms an anti-tilting surface for preventing the print papers from tilting.

In the case where the printing mechanism is hung vertically and the front end wall 16e of the print paper drawer 16 faces downward, even if the print papers have been added into the print paper drawer, the bottom of the laminated print papers is in contact with the anti-tilting surface which exerts an obliquely directed upwardly force on the print papers. A resultant force of the obliquely directed upwardly force and the gravity of the print papers is directed toward the bottom plate 16a of the print paper drawer so that the end of the print papers contacting the anti-tilting surface does not slip and tilt during printing while the smoothness in feeding the print paper to the printing head is not affected. Preferably, the second portion 16e₂ is bent at an angle α of 20° to 25° with respect to the first portion 16e₁.

Although the front end wall 16e may extend from the first sidewall 16b to the second sidewall 16c, the front end wall 16e preferably comprises front end wall portions that extend at a certain length from the first sidewall 16b and the second sidewall 16c respectively but do not extend from the first sidewall 16b to the second sidewall 16c. In this way, a space 16f is formed between the two front end wall portions so that the user may pick up and add the print papers with his hand through the space 16f.

A loading ramp 16g extending outwardly from a top edge of the first portion 16e₁ may also be disposed at the front end of the print paper drawer 16. The loading ramp 16g may be formed as an arcuate surface that gradually lowers toward the bottom plate 16a of the print paper drawer 16. When the printing mechanism is in a horizontally arranged state, the user simply places one end of the regularly folded print papers on the loading ramp 16g and then pushes slightly the other end, and the laminated print papers slide along the loading ramp 16g to a predetermined position in the print paper drawer under the effect of gravity without requiring the user to manually push the print papers completely into the print paper drawer.

The printing mechanism according to the present invention may further comprise a paper lifting mechanism 41 comprising a shaft 43 and two lifting bars 45 provided on the shaft 43. The two lifting bars 45 may rotate together with the shaft 43. It should be understood that the lifting bar 45 may be one or more. In the preferred embodiment, the shaft 43 and the lifting bars 45 are integrally formed by injection molding. The shaft 43 is rotatably provided at the front end of the print paper drawer 16. In the preferred embodiment, open rings 16h are formed at the front end of the paper drawer 16 and the shaft 43 can be snapped into the open rings 16h so that the shaft 43 is rotatably received in the open rings 16h. It should be understood that the shaft 43 may also be rotatably provided at the front end of the print paper drawer 16 by other suitable ways. The paper lifting mechanism 41 also comprises an operating handle 47 which may pass through a mounting plate of the frame 11 and be attached to the shaft 43. One end of a reset spring 49 is attached to the operating handle 47 and the other end is attached to the frame 11 or the print paper drawer 16. An opening 16i corresponding to the lifting bar 45 is formed in the bottom plate 16a of the print paper drawer 16. The opening 16i is used to accommodate the lifting bar 45 or allow the lifting bar 45 to pass therethrough.

In the existing printing mechanism, the user needs to manually grip the print paper from the gap between the print paper drawer and the print paper. However, this gap is generally very small so that it is very inconvenient for the user to replace the print paper. According to the present invention, when the user needs to replace the print papers or adjust the state of the print papers in the print paper drawer, the movable frame assembly on which the print paper drawer is mounted is firstly pulled outwardly from the housing of the medical monitoring device, the shaft 43 and the lifting bar 45 are driven to rotate by turning the operating handle 47 to rise the lifting bar 45 from the bottom plate 16a of the print paper drawer 16 so as to lift the print papers upwardly and outwardly, thereby facilitating the user to replace the print papers. A silicone sleeve 51 may be provided at the end of the lifting bar 45. The silicone sleeve 51 further drags the print papers toward the front end (exit) of the print paper drawer as the lifting bar 45 rotates, thereby enabling the print papers to be fed out. When the operating handle 47 is released, the operating handle turns back to an initial position under the action of the reset spring 49 and thus drives the lifting bars 45 to go back to the opening 16i or a position below the bottom panel 16a.

According to the present invention, by appropriately selecting the size of the length, width, and height of the print paper drawer, a balance between the compact position-limit of the print papers and the smoothness in loading and unloading of the print papers can be achieved, and a good balance between the compact position-limit of the print papers and the smoothness in feeding out the print paper can be achieved.

Although the present invention has been described in detail in connection with preferred embodiments, it should be understood that such detailed description is intended only to illustrate the present invention and does not constitute any limit to the present invention. For example, the first locking member may be provided as a shaft, the second locking member may be provided as a sleeve for receiving the shaft, and the shaft and the sleeve may engage releasably with each other by friction force. Thus, the scope of the present invention is defined by the technical solutions in the claims.

## Claims

1. A printing mechanism for a medical monitoring device comprising:
a movable frame assembly (9) comprising a frame (11); and
a print paper drawer (16) for holding the print papers, the print paper drawer (16) being mounted on the frame (11) to move along with the movable frame assembly;
wherein a front end wall (16e) of the print paper drawer (16) is formed as a curved wall comprising a first portion (16e₁) extending perpendicularly from a front end of a bottom plate (16a) of the print paper drawer (16) and a second portion (16e₂) extending from the first portion (16e₁) and bending towards a rear end of the print paper drawer (16) with respect to the first portion (16e₁), and the second portion (16e₂) forms an anti-tilting surface for preventing the print papers from tilting.

2. A printing mechanism for a medical monitoring device as claimed in claim 1, wherein the second portion (16e₂) is bent at an angle (α) of 20° to 25° with respect to the first portion (16e₁).

3. A printing mechanism for a medical monitoring device as claimed in claim 1, wherein the front end wall (16e) comprises front end wall portions that extend from a first sidewall (16b) and a second sidewall (16c) of the print paper drawer (16) respectively so that a space (16f) is formed between two front end wall portions.

4. A printing mechanism for a medical monitoring device as claimed in claim 1, wherein the print paper drawer (16) further comprises a loading ramp (16g) extending outwardly from a top edge of the first portion (16e₁), and the loading ramp (16g) is formed as an arcuate surface that gradually lowers toward the bottom plate (16a) of the print paper drawer (16).

5. A printing mechanism for a medical monitoring device as claimed in claim 1, wherein the printing mechanism further comprises a paper lifting mechanism (41) comprising a shaft (43) rotatably provided at a front end of the print paper drawer (16), a lifting bar (45) provided on the shaft (43) to rotate together with the shaft (43) and an operating handle (47) attached to the shaft (43) to drive the shaft (43) to rotate, and the lifting bar (45) is accommodated in an opening (16i) in the bottom plate (16a) or can rotate along with the shaft to pass through the opening (16i) in the bottom plate (16a).

6. A printing mechanism for a medical monitoring device as claimed in claim 5, wherein the paper lifting mechanism (41) further comprises a reset spring (49) which is attached to the operating handle (47) to cause the operating handle (47) to turn back to an initial position.

7. A printing mechanism for a medical monitoring device as claimed in claim 5, wherein a silicone sleeve (51) is provided at the end of the lifting bar (45).

8. A printing mechanism for a medical monitoring device as claimed in claim 1, wherein the printing mechanism further comprises:
a support plate (3);
a support arm (5) disposed on the support plate (3), the support arm (5) provided with a printing head thereon, the movable frame assembly (9) further comprising a rubber platen (21) mounted at a front end of the frame (11) adjacent to the outside of the medical monitoring device to be in contact with the printing head under pressure; and
a driving mechanism for driving the rubber platen (21) to rotate;
wherein the movable frame assembly (9) is movably disposed on the support plate (3) and between the support plate (3) and the support arm (5).

9. A printing mechanism for a medical monitoring device as claimed in claim 8, wherein the printing mechanism further comprises a locking mechanism comprising a pair of resilient clamping claws (37) disposed opposite to each other at a rear end of the frame (11) and a locking post (39) disposed on the support plate (3) correspondingly to the resilient clamping claws (37), the resilient clamping claws (37) clamp together, the resilient clamping claws (37) open under an external pushing force to receive the locking post (39) and clamp the locking post (39) tightly, and the resilient clamping claws (37) are disengaged from the locking post (39) under an external pulling force.

10. A printing mechanism for a medical monitoring device as claimed in claim 8, wherein the driving mechanism comprises a first pulley (23) fixedly mounted at one end of the rubber platen (21), an electric motor (25) mounted on the frame (11), a second pulley mounted on a rotating shaft of the electric motor (25), and a timing belt (27) for connecting the first pulley (29) and the second pulley so as to drive the rubber platen (21) to rotate when the electric motor (25) operates, and the electric motor (25) is disposed at a rear end of the frame (11) adjacent to the inside of the medical monitoring device.

11. A printing mechanism for a medical monitoring device as claimed in claim 8, wherein the support arm (5) is L-shaped, the support arm (5) is rotatably mounted on the support plate (3) at one end (5a) and provided with a printing head mounting portion (7) at the other end (5b), and the printing head is mounted on the printing head mounting portion (7).

12. A printing mechanism for a medical monitoring device as claimed in claim 11, wherein the frame (11) comprises a first mounting plate (13) and a second mounting plate (15) disposed opposite to each other, and a first connecting plate (17) and a second connecting plate (19) connecting fixedly the first mounting plate (13) and the second mounting plate (15) together, a first guide slot (29) extending along a longitudinal direction is formed in the first mounting plate (13) and a second guide slot (31) extending along the longitudinal direction is formed in the second mounting plate (15), and two ends (7a and 7b) of the printing head mounting portion (7) are movably supported in the first guide slot (29) and the second guide slot (31).

13. A printing mechanism for a medical monitoring device as claimed in claim 1, wherein the medical monitoring device is a fetal monitor.

14. A medical monitoring device, wherein the medical monitoring device comprises a printing mechanism for a medical monitoring device as claimed in any one of claims 1-13.

## Patentansprüche

1. Druckmechanismus für eine medizinische Überwachungsvorrichtung, der Folgendes umfasst:
eine bewegliche Rahmenanordnung (9), umfassend einen Rahmen (11); und
eine Druckpapierschublade (16) zum Aufbewahren der Druckpapiere, wobei die Druckpapierschublade (16) am Rahmen (11) befestigt ist, um sich zusammen mit der beweglichen Rahmenbaugruppe zu bewegen;
wobei eine vordere Endwand (16e) der Druckpapierschublade (16) als eine gekrümmte Wand ausgebildet ist, die einen ersten Abschnitt (16e₁), der sich senkrecht von einem vorderen Ende einer Bodenplatte (16a) der Druckpapierschublade (16) erstreckt, und einen zweiten Abschnitt (16e₂), der sich vom ersten Abschnitt (16e₁) erstreckt und sich in Richtung eines hinteren Endes der Druckpapierschublade (16) in Bezug auf den ersten Abschnitt (16e₁) biegt, wobei der zweite Abschnitt (16e₂) eine Kippschutzfläche bildet, um ein Kippen der Druckpapiere zu verhindern.

2. Druckmechanismus für eine medizinische Überwachungsvorrichtung nach Anspruch 1, wobei der zweite Abschnitt (16e₂) in einem Winkel (α) von 20° bis 25° in Bezug auf den ersten Abschnitt (16e₁) gebogen ist.

3. Druckmechanismus für eine medizinische Überwachungsvorrichtung nach Anspruch 1, wobei die vordere Endwand (16e) Vorderendwandabschnitte aufweist, die sich jeweils von einer ersten Seitenwand (16b) beziehungsweise einer zweiten Seitenwand (16c) des Druckpapierfachs (16) aus erstrecken, sodass zwischen zwei Vorderendwandabschnitten ein Raum (16f) gebildet wird.

4. Druckmechanismus für eine medizinische Überwachungsvorrichtung nach Anspruch 1, wobei die Druckpapierschublade (16) weiter eine Laderampe (16g) umfasst, die sich von einer Oberkante des ersten Abschnitts (16e₁) nach außen erstreckt, und die Laderampe (16g) als eine bogenförmige Fläche ausgebildet ist, die sich allmählich zur Bodenplatte (16a) der Druckpapierschublade (16) hin absenkt.

5. Druckmechanismus für eine medizinische Überwachungsvorrichtung nach Anspruch 1, wobei der Druckmechanismus weiter einen Papierhebemechanismus (41) umfasst, der eine drehbar an einem vorderen Ende der Druckpapierschublade (16) angeordnete Welle (43), eine auf der Welle (43) bereitgestellte Hebestange (45), die sich zusammen mit der Welle (43) dreht, und einen an der Welle (43) befestigten Bedienhebel (47) zum Antreiben der Welle (43) zum Drehen umfasst, und die Hebestange (45) in einer Öffnung (16i) in der Bodenplatte (16a) aufgenommen ist oder sich zusammen mit der Welle drehen kann, um durch die Öffnung (16i) in der Bodenplatte (16a) hindurchzugehen.

6. Druckmechanismus für eine medizinische Überwachungsvorrichtung nach Anspruch 5, wobei der Papierhebemechanismus (41) weiter eine Rückstellfeder (49) umfasst, die am Bedienhebel (47) befestigt ist, um zu bewirken, dass sich der Bedienhebel (47) zu einer Ausgangsposition zurückdreht.

7. Druckmechanismus für eine medizinische Überwachungsvorrichtung nach Anspruch 5, wobei am Ende der Hebestange (45) eine Silikonhülle (51) bereitgestellt wird.

8. Druckmechanismus für eine medizinische Überwachungsvorrichtung nach Anspruch 1, wobei der Druckmechanismus weiter Folgendes umfasst:
eine Stützplatte (3);
einen auf der Stützplatte (3) angeordneten Stützarm (5), wobei der Stützarm (5) mit einem Druckkopf versehen ist, wobei die bewegliche Rahmenanordnung (9) weiter eine Gummiplatte (21) umfasst, die an einem vorderen Ende des Rahmens (11) angrenzend an die Außenseite der medizinischen Überwachungsvorrichtung angebracht ist, um unter Druck mit dem Druckkopf in Kontakt zu stehen; und
einen Antriebsmechanismus zum Antreiben der Gummiplatte (21) zum Drehen;
wobei die bewegliche Rahmenanordnung (9) beweglich auf der Stützplatte (3) und zwischen der Stützplatte (3) und dem Stützarm (5) angeordnet ist.

9. Druckmechanismus für eine medizinische Überwachungsvorrichtung nach Anspruch 8, wobei der Druckmechanismus weiter einen Verriegelungsmechanismus, der ein Paar elastischer Klemmklauen (37) umfasst, die an einem hinteren Ende des Rahmens (11) einander gegenüberliegend angeordnet sind, und einen Verriegelungsbolzen (39), der auf der Stützplatte (3) entsprechend den elastischen Klemmklauen (37) angeordnet ist, umfasst, wobei sich die elastischen Klemmklauen (37) zusammenklemmen, wobei sich die elastischen Klemmklauen (37) unter einer äußeren Druckkraft öffnen, um den Verriegelungsbolzen (39) aufzunehmen und den Verriegelungsbolzen (39) fest zu klemmen, und die elastischen Klemmklauen (37) unter einer äußeren Zugkraft vom Verriegelungsbolzen (39) ausgerückt werden.

10. Druckmechanismus für eine medizinische Überwachungsvorrichtung nach Anspruch 8, wobei der Antriebsmechanismus eine erste Riemenscheibe (23), die an einem Ende der Gummiplatte (21) fest montiert ist, einen am Rahmen (11) montierten Elektromotor (25), eine zweite Riemenscheibe, die auf einer rotierenden Welle des Elektromotors (25) montiert ist, und einen Zahnriemen (27) zum Verbinden der ersten Riemenscheibe (29) und der zweiten Riemenscheibe, um die Gummiplatte (21) beim Betrieb des Elektromotors (25) in Rotation zu versetzen, umfasst und der Elektromotor (25) an einem hinteren Ende des Rahmens (11) angrenzend an die Innenseite der medizinischen Überwachungsvorrichtung angeordnet ist.

11. Druckmechanismus für eine medizinische Überwachungsvorrichtung nach Anspruch 8, wobei der Stützarm (5) L-förmig ist, der Stützarm (5) an einem Ende (5a) drehbar auf der Stützplatte (3) montiert ist und am anderen Ende (5b) mit einem Druckkopf-Montageabschnitt (7) versehen ist und der Druckkopf auf dem Druckkopf-Montageabschnitt (7) montiert ist.

12. Druckmechanismus für eine medizinische Überwachungsvorrichtung nach Anspruch 11, wobei der Rahmen (11) eine erste Montageplatte (13) und eine zweite Montageplatte (15) die einander gegenüberliegend angeordnet sind, und eine erste Verbindungsplatte (17) und eine zweite Verbindungsplatte (19) umfasst, welche die erste Montageplatte (13) und die zweite Montageplatte (15) fest miteinander verbinden, umfasst, in der ersten Montageplatte (13) ein erster Führungsschlitz (29), der sich entlang einer Längsrichtung erstreckt, ausgebildet ist und in der zweiten Montageplatte (15) ein zweiter Führungsschlitz (31), der sich entlang der Längsrichtung erstreckt, ausgebildet ist und zwei Enden (7a und 7b) des Druckkopf-Montageteils (7) beweglich in dem ersten Führungsschlitz (29) und dem zweiten Führungsschlitz (31) gelagert sind.

13. Druckmechanismus für eine medizinische Überwachungsvorrichtung nach Anspruch 1, wobei die medizinische Überwachungsvorrichtung ein Fetalmonitor ist.

14. Medizinische Überwachungsvorrichtung, wobei die medizinische Überwachungsvorrichtung einen Druckmechanismus für eine medizinische Überwachungsvorrichtung nach einem der Ansprüche 1-13 umfasst.

## Revendications

1. Mécanisme d'impression pour un dispositif de surveillance médicale comprenant :
un ensemble de cadre mobile (9) comprenant un cadre (11) ; et
un tiroir à papiers d'impression (16) destiné à contenir les papiers d'impression, le tiroir à papiers d'impression (16) étant monté sur le cadre (11) pour se déplacer avec l'ensemble de cadre mobile ;
dans lequel une paroi d'extrémité avant (16e) du tiroir à papiers d'impression (16) est formée comme une paroi incurvée comprenant une première partie (16e₁) s'étendant perpendiculairement à partir d'une extrémité avant d'une plaque inférieure (16a) du tiroir à papiers d'impression (16) et une seconde partie (16e₂) s'étendant à partir de la première partie (16e₁) et se pliant vers une extrémité arrière du tiroir à papiers d'impression (16) par rapport à la première partie (16e₁), et la seconde partie (16e₂) forme une surface anti-basculement pour empêcher le basculement des papiers d'impression.

2. Mécanisme d'impression pour un dispositif de surveillance médicale selon la revendication 1, dans lequel la seconde partie (16e₂) est pliée à un angle (α) de 20° à 25° par rapport à la première partie (16e₁).

3. Mécanisme d'impression pour un dispositif de surveillance médicale selon la revendication 1, dans lequel la paroi d'extrémité avant (16e) comprend des parties de paroi d'extrémité avant qui s'étendent respectivement d'une première paroi latérale (16b) et d'une seconde paroi latérale (16c) du tiroir à papiers d'impression (16) de sorte qu'un espace (16f) soit formé entre deux parties de paroi d'extrémité avant.

4. Mécanisme d'impression pour un dispositif de surveillance médicale selon la revendication 1, dans lequel le tiroir à papiers d'impression (16) comprend en outre une rampe de chargement (16g) s'étendant vers l'extérieur à partir d'un bord supérieur de la première partie (16e₁), et la rampe de chargement (16g) est formée comme une surface arquée qui descend progressivement vers la plaque inférieure (16a) du tiroir à papiers d'impression (16).

5. Mécanisme d'impression pour un dispositif de surveillance médicale selon la revendication 1, dans lequel le mécanisme d'impression comprend en outre un mécanisme de levage de papier (41) comprenant un arbre (43) prévu de manière rotative à une extrémité avant du tiroir à papiers d'impression (16), une barre de levage (45) prévue sur l'arbre (43) pour tourner avec l'arbre (43) et une poignée de commande (47) fixée à l'arbre (43) pour provoquer la rotation de l'arbre (43), et la barre de levage (45) est logée dans une ouverture (16i) de la plaque inférieure (16a) ou peut tourner avec l'arbre pour passer à travers l'ouverture (16i) de la plaque inférieure (16a).

6. Mécanisme d'impression pour un dispositif de surveillance médicale selon la revendication 5, dans lequel le mécanisme de levage de papier (41) comprend en outre un ressort de rappel (49) qui est fixé à la poignée de commande (47) pour faire revenir la poignée de commande (47) à une position initiale.

7. Mécanisme d'impression pour un dispositif de surveillance médicale selon la revendication 5, dans lequel un manchon en silicone (51) est prévu à l'extrémité de la barre de levage (45).

8. Mécanisme d'impression pour un dispositif de surveillance médicale selon la revendication 1, dans lequel le mécanisme d'impression comprend en outre :
une plaque de support (3) ;
un bras de support (5) disposé sur la plaque de support (3), le bras de support (5) étant muni d'une tête d'impression sur celui-ci, l'ensemble de cadre mobile (9) comprenant en outre un plateau en caoutchouc (21) monté à une extrémité avant du cadre (11) adjacente à l'extérieur du dispositif de surveillance médicale pour être en contact avec la tête d'impression sous pression ; et
un mécanisme d'entraînement pour faire tourner le plateau en caoutchouc (21) ;
dans lequel l'ensemble de cadre mobile (9) est disposé de manière mobile sur la plaque de support (3) et entre la plaque de support (3) et le bras de support (5).

9. Mécanisme d'impression pour un dispositif de surveillance médicale selon la revendication 8, dans lequel le mécanisme d'impression comprend en outre un mécanisme de verrouillage comprenant une paire de griffes de serrage élastiques (37) disposées face à face à une extrémité arrière du cadre (11) et un poteau de verrouillage (39) disposé sur la plaque de support (3) en regard des griffes de serrage élastiques (37), les griffes de serrage élastiques (37) se serrent ensemble, les griffes de serrage élastiques (37) s'ouvrent sous l'effet d'une force de poussée externe pour recevoir le poteau de verrouillage (39) et le serrer fermement, et les griffes de serrage élastiques (37) se désengagent du poteau de verrouillage (39) sous l'effet d'une force de traction externe.

10. Mécanisme d'impression pour un dispositif de surveillance médicale selon la revendication 8, dans lequel le mécanisme d'entraînement comprend une première poulie (23) montée de manière fixe à une extrémité du plateau en caoutchouc (21), un moteur électrique (25) monté sur le cadre (11), une seconde poulie montée sur un arbre de rotation du moteur électrique (25), et une courroie de distribution (27) pour relier la première poulie (29) et la seconde poulie afin de faire tourner le plateau en caoutchouc (21) lorsque le moteur électrique (25) fonctionne, et le moteur électrique (25) est disposé à une extrémité arrière du cadre (11) adjacente à l'intérieur du dispositif de surveillance médicale.

11. Mécanisme d'impression pour un dispositif de surveillance médicale selon la revendication 8, dans lequel le bras de support (5) est en forme de L, le bras de support (5) est monté de manière rotative sur la plaque de support (3) à une extrémité (5a) et est muni d'une partie de montage de tête d'impression (7) à l'autre extrémité (5b), et la tête d'impression est montée sur la partie de montage de tête d'impression (7).

12. Mécanisme d'impression pour un dispositif de surveillance médicale selon la revendication 11, dans lequel le cadre (11) comprend une première plaque de montage (13) et une seconde plaque de montage (15) disposées face à face, et une première plaque de liaison (17) et une seconde plaque de liaison (19) reliant de manière fixe la première plaque de montage (13) et la seconde plaque de montage (15), une première fente de guidage (29) s'étendant dans un sens longitudinal est formée dans la première plaque de montage (13) et une seconde fente de guidage (31) s'étendant dans le sens longitudinal est formée dans la seconde plaque de montage (15), et les deux extrémités (7a et 7b) de la partie de montage de tête d'impression (7) sont supportées de manière mobile dans la première fente de guidage (29) et la seconde fente de guidage (31).

13. Mécanisme d'impression pour un dispositif de surveillance médicale selon la revendication 1, dans lequel le dispositif de surveillance médicale est un moniteur fœtal.

14. Dispositif de surveillance médicale, dans lequel le dispositif de surveillance médicale comprend un mécanisme d'impression pour un dispositif de surveillance médicale selon l'une quelconque des revendications 1-13.
